# EUROPEAN PATENT APPLICATION

(11) **EP 4 501 203 A1**
(43) Date of publication of application: **05.02.2025**
(21) Application number: 23189325.6
(22) Date of filing: 02.08.2023
(51) Int. Cl.: A61B 1/00, A61B 1/04, G01N 21/64, G06T 7/12, G06T 11/00

(54) **DATA PROCESSING DEVICE AND COMPUTER-IMPLEMENTED METHOD FOR COMBINING A FLUORESCENCE EMISSION SIGNAL WITH AN EDGE DETECTION SIGNAL IN A MEDICAL OBSERVATION DEVICE**

(71) Applicant: Leica Instruments (Singapore) Pte Ltd, Singapore 608924 (SG)
(72) Inventor: THEMELIS, George, 608924 Singapore (SG)
(74) Representative: Grünecker Patent- und Rechtsanwälte PartG mbB

(57) **Abstract**

The invention relates to a data processing device (170) and a computer-implemented method for a medical observation device (100), such as a microscope or an endoscope, for observing an object (106) which contains a fluorophore (116, 118). The data processing device (170) is configured: to access input image data (120) representative of an image of the object (106), the input image data containing a reflectance signal (202), the reflectance signal being representative of light (198) reflected off the object, and a fluorescence emission signal (204), the fluorescence emission signal being representative of fluorescence emitted by the object; to apply an edge detection routine (148) to the reflectance signal (202) to generate an edge detection signal (600); and to generate a digital output image (160) from a combination of the edge detection signal and the fluorescence emission signal.

## Description

The invention relates to a data processing device and a computer-implemented method for a medical observation device, such as a microscope or endoscope. The medical observation device is configured for observing an object, which contains a fluorophore. The invention also relates to a medical observation device comprising a data processing device and to a method of operating a medical observation device, which method comprises the computer-implemented method.

A medical observation device may be used in surgery or in a laboratory context. In surgery, the object may be a part of the body of a patient, whereas in a laboratory context, the object may be an agglomeration of cells, a singular cell, or parts of a cell, e.g. in a biopsy. For detecting special properties of the object, fluorescence is often used. The object may naturally comprise one or more fluorophores-i.e. fluorescing materials-and/or one or more fluorophores may be artificially added to the object, e.g. by injection into a bloodstream or into a cell. Specific materials or parts of the object, which are of special interest, are marked and highlighted by the fluorescence emission of the fluorophore.

In surgery, for example, some fluorophores, such as 5-ALA, are used to mark tumors, whereas other fluorophores, such as ICG, are used to mark the blood flow. The autofluorescence of fluorophores naturally comprised in the object may be used to identify specific types of tissues.

The fluorescence of the one or more fluorophores is triggered by illuminating the object with light comprising or being confined to the fluorescence excitation spectrum of the at least one fluorophore.

The fluorescence from the at least one fluorophore is represented by a fluorescence emission signal that is recorded as part of input image data by the medical observation device. If only the fluorescence emission signal is displayed, a user may quickly recognize those parts of the object that are of interest. However, it is difficult for a user to establish quickly, where the fluorescing regions are located in the object or in the field of view of the medical observation device.

It is, therefore, an object of the invention to improve the images generated by the data processing device allowing a user to determine more reliably, where the fluorescing fluorophores are located in the object.

This object is achieved by a data processing device for a medical observation device, such as a microscope or an endoscope, for observing an object, which contains a fluorophore, wherein the data processing device is configured: to access input image data representative of an image of the object, the input image data containing a reflectance signal, the reflectance signal being representative of light reflected off the object, and a fluorescence emission signal, the fluorescence emission signal being representative of fluorescence emitted by the object; to apply an edge detection routine to the reflectance signal to generate an edge detection signal; and to generate a digital output image from a combination of the edge detection signal and the fluorescence emission signal.

The invention further relates to a computer-implemented method for processing a set of digital input images in a medical observation device, such as a microscope or endoscope, the method comprising the steps of: accessing input image data, representative of an image of the object, the input image data containing a reflectance signal, the reflectance signal being representative of light reflected off the object, and a fluorescence emission signal, the fluorescence emission signal being representative of fluorescence emitted by the object; applying an edge detection routine to the reflectance signal to generate an edge detection signal; assigning at least one pseudo-color to the fluorescence emission signal; and generating a digital output image from a combination of the edge detection signal and the fluorescence emission signal.

Including the edge detection signal in the digital output image provides a visual reference frame to facilitate the location of the fluorescence emitting parts of the object e.g. by a surgeon, without compromising detectability of the edge detection signal.

The solutions above can be further improved by one or more of the features that are described in the following. These features can be combined independently of one another. Further, each of the following features may be used equally for improving the data processing device and/or the computer-implemented method, even if the respective feature is described in the context of only the data processing device or only the computer-implemented method. For example, a feature or step that is described in the following in the context of the computer-implemented method only can be used to improve the data processing device generally in that the data processing device is configured to execute or perform this method feature or step. In particular, the data processing device may comprise a routine, which may be implemented in software, hardware, or a combination of software and hardware, which, upon execution, carries out the method feature or step. Conversely, a feature that is described only in the context of the data processing device may be used to improve the computer-implemented method.

According to one aspect, the input image data comprise one or more digital input images. The at least one digital input image may be a color input image or a monochrome image. The input image data may contain a plurality of input pixels. Each input pixel represents the light received from a specific location on the object. Each input pixel corresponds to a specific location on the object. If the input image data contain more than one digital input image, a specific location on the object may be represented by more than one input pixel. Input pixels that represent the same location on the object are termed "corresponding pixels" as is usual in the art.

The input pixels may be color pixels or monochrome pixels. A color input pixel comprises color space coordinates that define the color of the input pixel using, e.g., a color space. The color space coordinates represent the color appearance parameters, such as hue, lightness, brightness, chroma, colorfulness and saturation. If a tristimulus color space such as RGB is used, each input pixel comprises three color space coordinates R, G, B. The color space coordinate R defines the intensity of the red color band, the color space coordinate G defines the intensity of the green color band, and the color space coordinate B defines the intensity of the blue color band. For other color spaces, such as HSV, CIELAB or CIELUV, use other color space coordinates. A monochrome input pixel just indicates light intensity.

Throughout this text, if a digital input image is a color image, it is termed a "digital color input image". A multispectral or hyperspectral image is considered as a color image. If a digital input image is a monochrome image, it is termed "digital monochrome input image". If, in a specific context, it does not matter whether the digital input image is a color or a monochrome image, the generic term "digital input image" is used. A "digital input image" may thus be a "digital color input image" or a "digital monochrome input image". The same terminology is used for the digital output image.

Each digital input image may contain a plurality of in pixels. In a digital color input image, each input pixel may be a color pixel. In the digital fluorescence color output image, each pixel or, more specifically, each output pixel may be a color pixel. A color pixel contains a set of color space coordinates which, at least in combination, represent at least some of the color appearance parameters such as hue, lightness, brightness, chroma, colorfulness, etc. A monochrome image may represent only light intensity values, e.g., on a grey-scale.

In one specific example, the input image data may comprise at least one of: at least one digital color input image; at least one digital color input image and at least one digital monochrome input image; and at least two digital monochrome input images of the object. These are the respective sets of digital input images that may contain both a reflectance signal and a fluorescence emission signal that can be separated from one another reliably. It is, however, also possible that the input image set only includes a single digital monochrome input image which contains or consists of the specular reflection signal and the fluorescence emission signal.

If the input image data comprise a plurality of digital input images, it is preferred that each of the plurality of digital input images represents a different spectrum. In particular, the spectra of different digital input images of the plurality of digital input images do not overlap or at least have only a minimum overlap. Further, the spectra of the different digital input images of the plurality of digital input images may be complimentary.

The digital output image may comprise a plurality of output pixels. The digital output image may be a monochrome image or a color image. The output pixels may be monochrome or color. For each output pixel, there is at least one corresponding input pixel in the input image data.

According to one embodiment, the reflectance signal may contain a specular reflection signal, the specular reflection signal being representative of specular reflection off the object. The digital output image may be generated e.g. by the data processing device, from a combination of the extracted specular reflection signal and the fluorescence emission signal. Combining the extracted specular reflection signal with the fluorescence emission signal in a single image, the digital output image, provides the user with another positional reference frame that is easy to recognize. The user can quickly detect the specular reflections in a reflectance image of the object-or the object itself-and thus immediately recognize where the fluorescing areas are located on the object. The addition of just the specular reflection signal further does not overload the digital output image with signals, so that the details of the fluorescence emission signal are not outshone or overshadowed by other signals and just a minimum positional reference is given.

The reflectance signal, the specular reflection signal and the fluorescence emission signal may be considered as discrete and distinguishable signals that are superposed on one another in the input image data, for example in one or more digital input images. Each input pixel may represent a superposition of the signals received from a specific location on the object. Each of the reflectance signal, the specular reflection signal and the fluorescence emission signal represents itself a digital color or monochrome image of the object, which can be distinguished from another signal by at least one color appearance parameter.

The reflectance signal and the fluorescence emission signal may be contained together in their entirety in a single digital input image, preferably a single digital color input image. Alternatively, the reflectance signal and/or the fluorescence emission signal may be contained in different digital input images. For example, the reflectance signal may be contained in one digital input image, whereas the fluorescence emission signal may be contained in another digital input image.

According to another aspect, the reflectance signal and/or the fluorescence emission signal may be distributed across a plurality of digital input images. In this case, a digital input image may contain only a part of the reflectance signal and/or the fluorescence emission signal. If more than one signal is contained in a digital input image, this digital input image should be a color image so that the signals can be separated from one another.

If both the reflectance signal, either in its entirety or a part thereof, and the fluorescence emission signal, either in its entirety or a part thereof, are contained in a single digital input color image, the data processing device is preferably configured to extract the reflectance signal as contained in the digital input color image and/or the fluorescence emission signal as contained in the digital input color image. The data processing device may comprise a separation or extraction routine for extracting and/or separating the fluorescence emission signal and/or the reflectance signal from the remainder of the digital input image data or a digital input image comprised by the input image data.

The extraction of any signal, such as the reflectance signal, the fluorescence emission signal and/or the specular reflection signal from the input image data or a digital input image may involve a linear transformation and/or spectral unmixing. The linear transformation and the spectral unmixing are preferably carried out on a pixel-by-pixel basis. The linear transformation may comprise a matrix multiplication of an input pixel in a digital input image or of corresponding input pixels in different digital input images by a transformation matrix.

In the case, where a digital input image comprises both at least part of the fluorescence emission signal and at least part of the reflectance signal, the digital data processing device is preferably configured to separate the reflectance signal and/or the specular reflection signal contained in the digital input image from the fluorescence emission signal contained in the digital input image. The data processing device may comprise a specular reflection extraction routine for extracting the specular reflectance signal either from the extracted reflectance signal or directly from the input image data or the at least one digital input image that contains the specular reflection signal.

If parts of a signal, such as the reflectance signal, the specular reflection signal and/or the fluorescence emission signal are contained in a plurality of digital input images, the data processing device may be configured to combine the extracted parts of the respective signal. Such a combination may comprise a linear transformation, such as an addition, or alpha-blending of the parts. This allows to restore a more complete signal from its respective fragments that are contained in various digital input images.

For example, the input image data may comprise a first digital input image which contains part of the reflectance signal and at least part of the fluorescence emission signal and a second digital input image which contains an additional part of the reflectance signal. The data processing device may be configured to extract the part of the reflectance signal from the first digital input image and to extract the additional part of the reflectance signal from the second digital input image. The data processing device may further be configured to compute the reflectance signal from the part of the reflectance signal that is contained in the first digital input image and from the additional part that is contained in the second digital input image. The reflectance signal may be computed by combing the two parts as described above by using a linear transform and/or alpha blending.

According to another example, the second or a third input image may contain an additional part of the fluorescence emission signal. The data processing device may be configured to extract the part of the fluorescence emission signal from the first digital input image and to extract the additional part of the fluorescence emission signal from the second or third digital input image. The data processing device may further be configured to compute the fluorescence emission signal from the part of the fluorescence emission signal that is contained in the first digital input image and from the additional part that is contained in the second or third digital input image. The fluorescence emission signal may be computed by combing the two parts as described above by using a linear transform and/or alpha blending.

As a specular reflection corresponds to a total reflection of the light illuminating the object, a specular reflection may represent the brightest spots in a reflectance image of the object. Thus, according to one aspect, the data processing device may be configured to extract the specular reflection signal by extracting pixels from the input image data or a digital input image depending on the intensity of these pixels. The specular reflection signal may be extracted directly from the input image data. Alternatively, first the reflection signal may be extracted and then the specular reflection signal may be extracted from the reflectance signal.

In one variant, pixels are extracted from the input image data or a digital input image if their intensity is larger than a predetermined intensity threshold. Thus, only pixels having an intensity that exceeds the predetermined intensity threshold are considered in the specular reflection signal.

In order to obtain consistent results when extracting the specular reflection signal, it may be advisable to normalize the input image data, in particular at least those digital input images that contain the reflectance signal, prior to extracting the specular reflection signal.

As the specular reflection corresponds to a total reflection, the color of the specular reflection corresponds to the color of the light illuminating the object. Thus, according to another variant, the specular reflection signal may be extracted by extracting pixels from the input image data depending on their color. In particular, the color of a pixel of the input image data may be compared to a predetermined color, which may be stored in a memory of the data processing device. The predetermined color may correspond to the color of the illumination.

If the medical observation device has a tunable light source, the stored predetermined color may correspond to the respective current color of illumination in that the medical observation device may be configured to automatically update the stored predetermined color depending on the present color of the illumination.

The data processing device may, according to another aspect, be configured to extract the specular reflection signal by extracting pixels having a color that is contained in a predetermined set of colors. The predetermined set of colors may be representative of the current color of the light source illuminating the object. The predetermined set of colors may be automatically updated by the medical observation device depending on the current illumination. Using a predetermined set of colors instead of a single predetermined color makes the extraction of the specular reflection signal more robust. The predetermined set of colors or the single predetermined color may be obtained by calibration. The predetermined set of colors may correspond to a region in a color space, which region may be contiguous.

The data processing device may be configured to color the extracted specular reflection signal in the predetermined color, wherein at least one color appearance parameter of the predetermined color of the specular reflectance signal in the digital output color image may be dependent on the intensity of the specular reflectance signal in the corresponding pixel. Thus, the specular reflections obtain their natural color in the digital output image.

The extraction of pixels depending on their intensity may be combined with the extraction of pixels depending on their color. This makes the extraction of the specular reflection signal more robust.

In some embodiments, the input image data may comprise a digital fluorescence input image, which represents the fluorescence emission of the object and may contain part of the reflectance signal, and a digital reflectance input image which comprises at least the major part or of the reflectance signal. The digital fluorescence input image may be a digital input image which represents light recorded in discrete narrow-band passbands, which may be located in the fluorescence emission spectrum or spectra of the at least one fluorophore. The digital reflectance input image may be a digital input image which represents light recorded in at least one wide-band passband. In particular, the reflectance signal as contained in the digital reflectance image may represent a white-light reflectance image of the object.

The reflectance signal may represent light in the fluorescence excitation spectrum of the fluorophore, the fluorescence emission of which is represented in the fluorescence emission signal. The low frequency end of the fluorescence excitation spectrum may overlap the high frequency end of the fluorescence emission spectrum as represented in the fluorescence emission signal. Thus, illumination of the object which is used for triggering fluorescence, may create reflections, i.e. parts of the reflectance signal that are recorded in the fluorescence emission spectrum.

The digital fluorescence input image and the digital reflectance input image may be color images.

The reflectance signal may represent reflected light in the NIR (near infrared) range, i.e., have wavelengths larger than 700 nanometers and preferably smaller than 1000 nanometers. Such a part of the reflectance signal may have been recorded in a passband which preferably is restricted to the NIR band. The reflected light in the NIR range represents a good approximation to light reflected in the visible red range. The part of the reflectance signal located in the NIR range may thus be used to supplement the red color where a reflectance image could not be recorded in the visible red range. This may be the case if the fluorescence emission of the at least one fluorophore is located in the visible red range.

In one embodiment, the input image date may contain a first (part of the) reflectance signal and a second (part of the) reflectance signal, wherein the first reflectance signal represents light reflected off the object and having a first type of polarization, and the second part of the reflectance signal represents light reflected off the object and having a second type of polarization, wherein the second type of polarization is different from the first type of polarization. In one example, the first reflectance signal may be a reflectance signal without a specular reflection signal and the second reflectance signal may be a reflectance signal with the specular reflection signal.

According to one aspect, the first type of polarization may comprise unpolarized light. According to another aspect, the second type of polarization may comprise linearly or circularly polarized light.

The data processing device may be configured to obtain the specular reflection signal from a combination of the first reflectance signal and the second reflectance signal. The combination of the part of the reflectance signal in the first digital input image and the additional part of the reflectance signal may comprise an application of a linear transform, in particular a subtraction, to the first and second reflectance signal. The subtraction may be performed pixelwise by the data processing device.

The first and the second reflectance signal may be contained in a single digital input image or the first reflectance signal may be contained in a first digital input image and the second reflectance signal may be contained in a second digital input image. If the first and second reflectance signals are contained in a single digital input image, they may constitute a first and a second image channel, respectively.

The edge detection signal may be combined with the fluorescence emission signal with or without the extracted specular reflection signal and/or with the reflectance signal in the digital output image.

Generating the edge detection signal may comprise applying an edge detection routine to the reflectance signal. The edge detection routine may be part of the data processing device and be implemented in hardware, software, or a combination of hardware and software.

The edge detection routine may be a routine, which is available from image processing libraries. The edge detection routine may be isotropic or anisotropic. The edge routine may be applied to one or more color space coordinates of the reflectance signal individually or simultaneously.

The edge detection signal may be monochrome in the digital output image or it may be colored in a false color, which is preferably different from a color of the fluorescence emission signal. The intensity of the false color at an output pixel may depend on the intensity of the edge detection signal at a corresponding pixel. Alternatively, the edge detection signal may be colored in a pseudocolor, where the color at an output pixel may depend on the intensity of the edge detection signal at a corresponding pixel. The pseudocolors used for the edge detection signal are preferably different from the color of the fluorescence emission signal in the digital output image.

According to a further embodiment, the data processing device may be configured to generate the digital output image from a combination of at least two signals of the group of signals containing the specular reflection signal, the reflectance signal without the specular reflection signal, the reflectance signal and the fluorescence emission signal, the combination depending on a user selection signal.

Further, the data processing device may be configured to combine at least two signals of the group of signals containing the specular reflection signal, the reflectance signal without the specular reflection signal, the reflectance signal, the edge detection signal and the fluorescence emission signal, wherein a ratio of the intensities of the signals combined in the digital output image depends on the user selection signal.

The above configurations provide a plurality of different viewing modes, where a user is able to select the signals and optionally their intensities relative to one another in the digital output image.

For example, the digital processing device may be configured to change the ratio of the intensity of the specular reflection signal to the intensity of the fluorescence emission signal in the digital output image depending on the user input signal.

The digital processing device may also be configured to change the ratio of the intensity of the edge detection signal to the intensity of the fluorescence emission signal.

The digital processing device may be configured to change the ratio of the intensity of the reflectance signal not containing the specular reflection signal and the intensity of the fluorescence emission signal.

The digital processing device may further be configured to change the ratio of the intensity of the specular refection signal to the intensity of the edge detection signal. The ratio of intensities, as mentioned above, may be adjusted by changing either the intensity of just one of the two signals mentioned respectively, or jointly changing both intensities of these signals.

For providing the user selection signal, the medical observation device may comprise a mode selector, such as a physical button, knob or dial, and/or a widget.

The medical observation device may comprise a data processing device in any of the embodiments described above and at least one camera, which is adapted to record the input image data.

The at least one camera may be at least one color camera, or at least one color camera and at least one monochrome camera, or at least two monochrome cameras. A multispectral camera or a hyperspectral camera is considered as a color camera. Each camera of the medical observation device may be configured to record a separate digital input image that is contained in the input image data.

If the medical observation device comprises a plurality of cameras, each camera of the plurality is preferably configured to record a digital input image in a different spectrum. The different spectra are preferably non-overlapping and/or complimentary. At least one camera may be configured to record in the NIR range.

The medical observation device may comprise an observation filter assembly, which is located in an optical path between the object and the at least one camera. The optical filter assembly comprises one or more passband filters having one or more passbands. At least one passband comprises, consists of, or is located in the fluorescence emission spectrum of the at least one fluorophore.

At least one camera may be configured to resolve polarization, e.g., by comprising an on-chip polarizer. Such a camera may provide a digital input image having two image or-here synonymously-polariza-tion channels, each polarization channel representing an image of the object in differently polarized light. One of these image channels may, for example, comprise the reflectance signal without the specular reflection signal and the other image channel may comprise the reflectance signal with the specular reflection signal.

At least one camera may be a digital reflectance camera, which is adapted to record at least part of the reflectance signal. The digital reflectance camera may, in particular, be adapted to record a digital color input image, which represents a white-light reflectance image of the object.

At least one camera may be a fluorescence camera, which is adapted to record the fluorescence emission signal. The fluorescence camera may further be configured to record part of the reflectance signal in the NIR range. The part of the observation filter assembly that is located between the object and the fluorescence camera may comprise at least one passband, which comprises, consists of or is located in the fluorescence emission spectrum of at least one fluorophore.

The digital reflectance camera may be configured to record a larger number wavelengths in the visual light range than the digital fluorescence camera. The digital reflectance camera may record light in a passband which is wider than any passband of the digital fluorescence camera. The visual light range may extend from 380 nm to 700 nm.

A method for using a medical observation device in one of the embodiments described above may comprise the steps of recording the input image data and carrying out the computer-implemented method in any of the above embodiments.

For generating the digital output image by a combination of signals, a signal/image combination routine may be comprised by the data processing device. The combination of signals may comprise the combination of at least two signals of the group of signals containing the reflectance signal, the specular reflection signal and the fluorescence emission signal. The combination of signals may involve a pixelwise addition of the color space coordinates of the corresponding pixels in the respective signals that are to be combined in the digital output image. Alternatively or cumulatively, the combination of signals may involve alpha blending and/or a linear transformation, such as multiplying the color space coordinates of all corresponding pixels by a transformation matrix.

The initially mentioned object is also achieved by a method for using a medical observation device, such as an endoscope or microscope, wherein the method comprises the steps of recording the input image data and carrying out the computer implemented method in any of the above-described configurations.

Finally, the invention is also concerned with a computer program product and/or a computer-readable medium comprising instructions, which when executed by a computer causes the computerto carry out the computer implemented method in any of the above configurations.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

In the following, the invention is described exemplarily with reference to various examples and with reference to the drawings. The combination of features that are described and/or shown in the drawings and/or examples is not to be considered as limiting. For example, features may be omitted from an embodiment if it has a technical effect e.g. as explained above, that is not needed in a specific application. Conversely, a feature described above that are not part of an embodiment described below may be added if the technical effect associated with this particular feature is beneficial in a specific application.

Throughout the description and the drawings, the same reference numerals are used for elements that correspond to each other with respect to function and/or structure.

In the drawings,
- Fig. 1: shows a schematic representation of a medical observation device;
- Fig. 2: shows a schematic representation of the generation of a digital output image from a specular reflection signal and a fluorescence emission signal;
- Fig. 3: shows a schematic representation of an extraction of a specular reflection signal from a digital input image and the generation of a digital output image from a fluorescence emission signal and the extracted specular reflection signal;
- Fig. 4: shows a schematic representation of an extraction of a specular reflection signal using color space coordinates;
- Fig. 5: shows a schematic representation of an extraction of a specular reflection signal using reflection signals representing differently polarized light;
- Fig. 6: shows a schematic representation of combining a fluorescence emission signal with an edge detection signal;
- Fig. 7: shows a schematic representation of coloring an edge detection signal and/or a fluorescence emission signal and combining them;
- Fig. 8: shows a schematic representation of various combinations of different signals;
- Fig. 9: shows a schematic representation of different display modes of a medical observation device;
- Fig. 10: presents a schematic overview of the steps for generating a digital fluorescence color output image;
- Fig. 11: shows a schematic representation of a generic medical observation device.

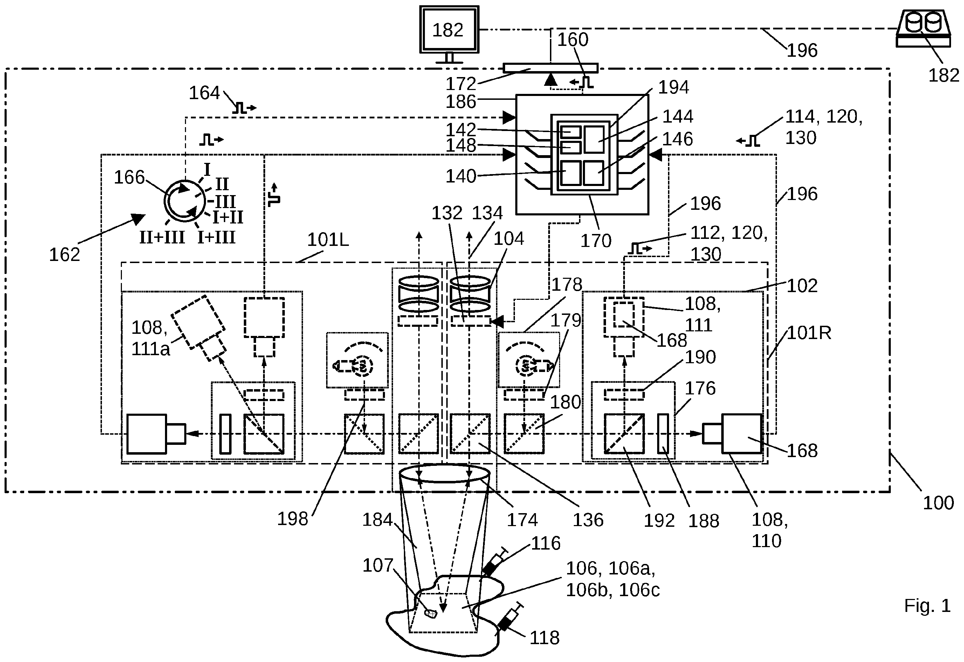

Fig. 1 shows schematically a medical observation device 100. The medical observation device 100 may be a microscope or an endoscope, the difference between a microscope and an endoscope being primarily that, in an endoscope (not shown), an object 106 is viewed through optical fibers that are brought into vicinity of the object 106 to be investigated, e.g. by insertion into a body containing the object, whereas, in a microscope, an objective 174 is directed onto the object. Although the medical observation device of Fig. 1 is a microscope, the following description also applies to an endoscope.

The medical observation device 100 may be a medical observation device used in surgery. The medical observation device 100 may also be a medical observation device used in a laboratory, such as a laboratory microscope. The object 106 to be investigated may consist of or comprise biological tissue 107. The object 106 may be a part of a patient's body that is located within the field of view of the medical observation device 100.

The object 106 may contain one or more fluorophores 116, 118. At least one of these fluorophores 116 may be a fluorophore that is naturally contained in the object. For example, bone and blood naturally contain fluorophores. Alternatively or cumulatively, at least one fluorophore 118 may be artificially added to the object 106, e.g. by injecting it into the biological tissue 107. Examples of fluorophores 118 that may be artificially added to the object 106 are ICG, fluorescein and/or 5-ALA.

The medical observation device 100 as shown is a fluorescence imaging device. Thus, the medical observation device is configured to view and preferably also excite the fluorescence of the one or more fluorophores 116, 118.

The medical observation device 100 may be a stereoscopic device as is exemplarily shown in Fig. 1. It may thus comprise two identical subassemblies 101L and 101R for each of the two stereoscopic channels. As the two subassemblies 101L, 101R are identical with respect to function and structure, the following description focuses on the right subassembly 101R, but applies identically to the left stereoscopic channel 101L.

The medical observation device 100 may alternatively be a monoscopic device. In this case, only one of the two subassemblies 101L, 101R may be present. For a monoscopic medical observation device 100, the following description therefore applies as well.

The medical observation device 100 in operation provides input image data 120. The input image data 120 are representative of an imaged scene, i.e. the part of the object that is within the field of view 184 of the medical observation device. The medical observation device 100 is configured to record in the input image data 120 the fluorescence of the at least one fluorophore 116, 118.

The input image data 120 may comprise one or more different digital input images 130. A digital input image 130 may correspond to a frame in a video stream or sequence. If the input image data 120 contain a plurality of digital input images 130, the different digital input images 130 should contain different spectral information. In such a case, each digital input image 130 of the input image data may be recorded at different wavelengths, preferably with no or, synonymously, minimum spectral overlap. Preferably, the spectra, in which the different digital input images 130 of the input image data 120 are recorded, are complementary and/or non-overlapping.

For generating the input image data 120, the digital imaging system 102 may comprise one or more digital cameras 108. The number of digital input images 130 contained in the input image data 120 may depend on and/or correspond to the number of cameras 108 used for generating the input image data 120. Depending on the type and setting of a respective digital camera 108, a digital input image 130 may be a color image or a monochrome image.

The input image data 120 contain a fluorescence emission signal that is representative of fluorescence emitted by the object 106, in particular by the at least one fluorophore 116, 118 contained therein. The fluorescence emission signal may does not need to contain all wavelengths in which the object 106 emits fluorescence; it may contain only a part of the fluorescence emitted by the at least one fluorophore 116, 118, e.g. the fluorescence that is emitted in one or more discrete passbands overlapping the fluorescence spectrum.

The medical observation device 100 is further configured to record a reflectance signal, which is representative of light reflected off the object 106 and also contained in the input image data 120. The reflected light may have been generated by white-light illumination, e.g. using a standard illuminant, or by illumination comprising or consisting of the fluorescence excitation spectrum of the at least one fluorophore 116, 118.

The reflectance signal contains or may consist of a specular reflection signal that represents specular reflections off the object 106.

The reflectance signal may, in one embodiment, comprise or consist of other a first reflectance signal without specular reflections and a second reflectance signal containing specular reflections. The reflectance signal without specular reflections may be contained in a different digital input image 130 than the reflectance signal with specular reflection. In particular, the reflectance signal with specular reflection may represent light with other polarization characteristics than the reflectance signal without specular reflection.

A single digital input image 130 may contain one or more signals, some signals may be contained in their entirety in the digital input image 130, while only parts other signals may be contained. If a digital input image 130 contains more than one signal, it is preferably a digital color input image 130 so that the different signals may be distinguished from one another e.g. by their spectral signatures. For example, a digital input color image 130 may contain a part of or the entire reflectance signal and a part of or the entire fluorescence emission signal, the rest of the reflectance signal and/or the fluorescence emission signal being contained in another digital input image 130. According to another example, the reflectance signal and the fluorescence emissions signal may be contained in different digital input images, respectively, which then may be digital input color or monochrome images 130.

Just by way of example, the digital imaging system 102 may comprise as digital cameras 108 a digital reflectance camera 110 and one or more digital fluorescence cameras 111, 111a. A second (or third) digital fluorescence camera 111a is optional. In Fig. 1, the second digital fluorescence camera 111a is only shown in the left stereoscopic channel 101L, but of course may also be present in the right stereoscopic channel 101R. Alternatively, the digital fluorescence camera of one stereoscopic channel may be used as the (first) digital fluorescence color camera 111 and the digital fluorescence camera of the other stereoscopic channel may be used as the second fluorescence camera 111a. The cameras 110, 111, 111a may each be a color camera or a monochrome camera. A multispectral camera or a hyperspectral camera is considered as a color camera.

The digital reflectance camera 110 is configured to record a digital reflectance input image 114, i.e. a digital input image 130, which is representative of the reflectance of the object 106 and thus may comprise all or at least a major part of the reflectance signal. The digital reflectance camera 110 is preferably configured to record a digital input image 130 in a wide spectral range within the visible light spectrum. Thus, the digital input image 130 recorded by the digital reflectance camera may represent closely the natural colors of the object 106. This is important if the digital reflectance camera 110 is used to provide the user with an image of the object which should come as close as possible to the human perception of the object. The digital reflectance camera 110 may be a CCD, CMOS or multispectral or hyperspectral camera.

The digital input image 130 recorded by the digital reflectance camera 110 may contain also at least part of the specular reflectance signal and/or the fluorescence emission signal.

The digital reflectance camera 110 may be configured to resolve the polarization of the incoming light, e.g., by comprising an on-chip polarizer 168. In such a case, the digital reflectance camera 110 may record both a reflectance signal with the specular reflection signal and a reflectance signal without the specular reflection signal.

The fluorescence camera 111 may be configured to record the digital fluorescence image only in one or more narrow spectral bands. These narrow spectral bands should overlap or contained within the fluorescence spectrum or spectra of the one or more fluorophores 116, 118 of which fluorescence is to be recorded. Preferably, the fluorescence spectra of the different fluorophores 116, 118 are at least partly separate, preferably completely separate, i.e. non-overlapping, so that the fluorescence camera 111 may record a digital color input image 130 representing two separate fluorescence bands that are spaced from one another.

Alternatively, if two or more fluorescence cameras 111, 111a are provided, each fluorescence camera 111, 111a preferably captures the fluorescence emission of a different fluorophore 116, 118, thus providing two digital fluorescence input images 112. The fluorescence emission signal may comprise or consist of the fluorescence of only one fluorophore 116, 118, although the fluorescence of more than one fluorophore 116, 118 was recorded, or if may comprise or consist of the fluorescence of more than one fluorophore 116, 188..

Any fluorescence camera 111, 111a may also capture part of the reflectance signal in its respective digital color input image 130. For example, a part, in particular a low-frequency end, of the excitation spectrum used for triggering the fluorescence of a fluorophore 116, 118 may overlap the fluorescence spectrum of this fluorophore and then be recorded as the or part of the reflectance signal together with the fluorescence emission. Preferably, the intensity of this reflectance signal is comparable to the intensity of the fluorescence so that the reflectance signal does not outshine the fluorescence emission.

The at least one fluorescence camera 111, 111a may be a monochrome camera, a CCD, CMOS or multispectral or hyperspectral camera. Preferably, the reflectance camera 110 and the at least one fluorescence color camera 111 are of the same type, although this is not necessary.

The at least one fluorescence camera 111, 111a may be configured to resolve polarization, e.g., by including an on-chip polarizer 168.

Any combination of the cameras 110, 111 and 111a may be functionally combined into a single multi-spectral or hyperspectral camera. The multispectral or hyperspectral then represents the function of one or more of the cameras 110, 111 and 111a. A digital color input image 130 recorded by a multispectral or hyperspectral camera may represent a plurality of digital input images 130 and may contain all of the fluorescence emission signal and all of the reflectance signal.

The respective fields of view 184 of the cameras 108 are preferably aligned or even coinciding and coaxial. It is preferred that the cameras 108 provide the identical field of view 184 with the identical perspective and focal length. This results in geometrically identical representations of the object 106 in the images 112, 114 generated by the different cameras 108. The cameras 108 may use the same objective 174. Preferably, the cameras 108 have the same resolution and/or number of pixels and/or aspect ratio.

If a match of the perspectives and field of view cannot be generated optically, it may be generated by image processing by applying a matching or registering routine to the digital input images 130, as is explained further below.

It is preferred that the cameras 108 are operated synchronously. Specifically, the exposure times may be synchronized. Thus, the medical observation device 100 may be configured to generate the digital input images 130 at the same time.

Preferably, the gain of the cameras 108 is synchronized, i.e. adjusted in the cameras 108 at the same time. Moreover, the ratio of the gain applied in one camera 110 to the gain applied in any other camera may remain constant, even if the gain is changed in any one camera 108. The gamma correction and color adjustment or white balance may be switched off or kept constant.

For separating the light recorded in one digital input image 130, e.g. the digital reflectance input image 114, from the spectrum recorded in another digital input image 130, e.g. the at least one digital fluorescence input image 112 an optical color-separation assembly 176 may be provided. The color-separation assembly 176 is used for optically separating the light spectra reaching the various cameras 108.

The color-separation assembly 176 may comprise optical elements such as a beam splitter 192, which may be dichroic. The color separation assembly 176 may further or alternatively comprise an optical observation filter set 188 and/or an optical fluorescence filter set 190. The optical observation filter set 188 and the fluorescence-filter set 190 may be part of an optical filter assembly 187.

The fluorescence filter set 190 is preferably configured to transmit light comprised in the fluorescence emission spectrum or spectra of the one or more fluorophores 116, 118 and to block light outside the fluorescence spectrum or spectra.

The fluorescence filter set 190 may comprise one or more passbands and it may comprise one or more optical filters. Each passband should overlap the fluorescence emission spectrum of a respective fluorophore 116, 118 of which the fluorescence is to be recorded. As the fluorescence filter set 190 is in the light path between the beam splitter 192 and the fluorescence color camera 111, only the wavelengths in the passbands of the fluorescence filter set 190 are transmitted to the fluorescence camera color 111.

If two fluorescence cameras 111, 111a are used to capture different fluorescence emission spectra, the fluorescence filter set 190 may comprise a different optical band-pass filter in front of each of the fluorescence color cameras 111, 111a. The pass-band of one band-pass filter may be contained in the fluorescence-emission spectrum of one fluorophore 116, whereas the pass-band of the other band-pass filter may be contained in the fluorescence-emission spectrum of another fluorophore 116, 118 in the object 106.

The observation filter set 188 is preferably configured to block light in the fluorescence spectrum or spectra of the one or more fluorophores 116, 118. The observation filter set 188 may also be configured to block light in the fluorescence-excitation spectrum.

The observation filter set 188 is preferably configured as a band-stop filter, of which the stop bands correspond to or at least contain the passbands of the fluorescence filter set 190. The observation filter set 188 is located in the light path between the beam splitter 192 and the reflectance camera 110. Thus, the reflectance camera 110 records only wavelengths that are outside the stop-bands of the observation filter set 188 and therefore also outside of the pass-bands of the fluorescence filter set 190 of the fluorescence filter set 190.

Any one of the observation filter set 188 and the fluorescence filter set 190 may comprise a tunable filter.

If the beam splitter 192 is a dichroic beam splitter, at least one of the filter sets 188, 190 may be omitted as the optical spectral filtering in this case is already integrated in the dichroic beam splitter. The above description of the passbands and stopbands then should apply mutatis mutandis to the dichroic beam splitter 192.

The medical observation device 100 may further comprise an illumination assembly 178, which is configured to illuminate the object 106 preferably through the objective 174 through which the imaging system 102 records the at least one digital image 112, 114.

The illumination assembly 178 may be configured to selectively generate white-light, i.e. light that is evenly distributed across the entire visible spectrum, and fluorescence-excitation light, which contains light only in wavelengths that stimulate fluorescence of the at least one fluorophore 116, 118. The illumination light generated by the illumination assembly 178 may be fed into the objective 174 using an illumination beam splitter 180.

The illumination assembly 178 may be configured to generate illumination light simultaneously in a plurality of discrete, in particular narrow-band, wavelength bands. These wavelength bands may comprise any of or any combination of the following wavelength bands.

One such discrete wavelength band may be entirely located in the fluorescence-excitation spectrum of a fluorophore 116. Another such wavelength band may be entirely located in the fluorescence-emission spectrum of another fluorophore 118. Another such wavelength band may be limited to wavelengths larger than 700 nm, e.g. up to 1000 nm, and be entirely located in the NIR (near infrared) range. The NIR wavelength band may be used as part of a reflectance signal to represent the red component, in particular the R component of an RGB digital color input image 130.

The simultaneous illumination of the object with any of the discrete wavelength bands as described above may be accomplished by a light source 199, e.g. a tunable light source such as a light source comprising a plurality of LEDs in different colors, in particular in different primary colors, which is configured to generate light in these wavelength bands simultaneously. Alternatively or additionally, the wavelength bands may be generated by using an illumination filter 179 having multiple pass-bands, wherein the pass-bands preferably correspond to the above wavelength bands. If such an illumination filter 179 is used, the light source 199 may generate white-light which is then filtered by the illumination filter 179 so that only the light in the pass-bands illuminates the object 106.

The illumination filter 179 may be provided depending on the at least one fluorophore, of which fluorescence is to be triggered, and its specific excitation spectrum. For example, if 5-ALA is used as a fluorophore, the illumination filter may have a transmission of 90 % to 98 % up to wavelengths of 425 nm, a transmission between 0.5 % and 0.7 % in wavelengths between 450 nm and 460 nm, a transmission of not more than 0.1 % between 460 nm and 535 nm and of practically zero for wavelengths above 535 nm. The illumination filter 179 may be configured for pass-through of NIR light. For example, the illumination filter 179 may comprise a pass-band in the NIR. The illumination filter 178 may further comprise a passband, which is preferably entirely located in the fluorescence-excitation spectrum of another fluorophore.

The medical observation device 100 may be adjusted to a different fluorophore or set of fluorophores by re-configuring the color-separation assembly 176, e.g. by exchanging its optical elements, such as the filters set 190 and/or 192, or the dichroic beam splitter 180.

Using the observation filter system 188 as described above, the digital reflectance camera 110 may be used to record at least part of the fluorescence emission signal if the respective wavelengths pass through the pass-bands of the observation filter system 188. In such a case, the illumination of the object 106 should preferably not contain the fluorescence emission wavelengths, as the intensity of the fluorescence is often low, which may make the fluorescence signals harder to detect, as the reflected light outshines fluorescence. The same holds mutatis mutandum for the reflectance signal and the at least one digital fluorescence camera 111, 111a.

The input image data 120 are processed by a data processing device 170. The data processing device 170 may be an integral part of the medical observation device 100. In one example, it is a processor which is embedded in the medical observation device and also used as a controller for controlling the hardware of the medical observation device 100, such as the brightness and/or spectral emission of the light source 199 and/or any objective of the medical observation device 100 and/or any actuators of the medical observation device 100. In another example, the data processing device 170 is part of a general computer, which is connected to the medical observation device for unidirectional, or bidirectional data transfer by wire or wirelessly.

The data processing device 170 may be a hardware module, such as a microprocessor, or a software module. The data processing device 170 may also be a combination of both a hardware module and a software module, for example by using software modules that are configured to be run on a specific processor, such as a vector processor, a floating point graphics processor, a parallel processor and/or on multiple processors. The data processing device 170 may be part of a general-purpose computer 186, such as a PC. In another embodiment, the data processing device 170 is an embedded processor of the medical observation device 100.

The data processing device 170 is configured to access the input image data 120, e.g. in the form of one or more digital input images 130, such as the digital reflectance input image 114 and the digital fluorescence image 112. The data processing device 170 may be configured to retrieve the digital input images 130 from a memory 194 and/or directly from the at least one camera 108. The memory 194 may be part of the data processing device 170 or reside elsewhere in the medical observation device 100.

The data processing device 170 is further configured to compute a digital reflectance output image 160 from the input image data 120, specifically from the reflectance signal and the fluorescence-emission signal contained in the input image data 120.

The digital output image 160 may be a color image, which is represented in a color space. The color space of the digital color output image 160 may be different from the color space of any digital color input image 130 that is contained in the input image data 120. Preferably, however, the color space of the digital color output image 160 is the same color space as that of any digital color input image 130. Alternatively, however, the digital output image 160 may be a monochrome image. A digital monochrome output image 160 represents only light intensity as brightness.

If the fluorescence emission signal and/or the reflectance signal need to be processed separately, the respective signal needs to be separated or extracted from the input image data 120.

Extraction of a signal is straightforward if a digital input image consists of a single signal only, e.g., of only the fluorescence emission signal or the reflectance signal. Extracting the signal from such a digital input image 130 simply corresponds to using the respective digital input image 130 as the signal it consists of.

If, however, any two signals of the group of signals containing the fluorescence emission signal the reflectance signal and the specular reflection signal are contained in a single digital input image-which in this case should be a color image-they need to be separated from one another to be processed separately. To achieve this, the digital processing device 170 is configured to separate the fluorescence emission signal, the reflectance signal and/or the specular reflection signal from one another. More specifically, the digital processing device 170 may comprise a separation or extraction routine 140 that is configured to perform this separation if applied to one or more digital input images 130. The separation or extraction routine 140 may be stored in a memory 194 of the digital processing device 170. The separation or extraction routine 140 may comprise an unmixing routine 142 e.g. for spectrally unmixing the signals. For extracting the specular reflection signal a specular reflection extraction routine 146 may be comprised by the separation or extraction routine 142.

The extracted and separately processed signals may be combined after being processed in the digital output image 160 using a signal/image combination routine 144. The signal/image combination routine 144 may treat the extracted signals as images.

To detect contours of anatomical features of the object 106, an edge detection routine 148 may be applied to the reflectance signal, in particular the reflectance signal without the specular reflection signal. The edge detection routine 148 may also be contained in the data processing device 170, e.g., in the memory 194.

Any of the routines 140 to 148 may be a software routine, a routine implemented in hardware, or a routine in which software and hardware components are combined.

The medical observation device 100 may comprise a user input device 162, which is configured to be operated by a user. Upon operation, the user input device 162 may generate a user selection signal 164, which may be communicated to the digital processing device 170. The user input device 162 may e.g. be a physical button, dial, slide or lever, or a widget that represents a physical button, dial, slide, lever, or widget.

By operating the user input device 162, the user may determine which signal or combination of signals is to be contained in the digital output image 160 and/or in which intensity a signal is contained in the digital output image 160. These different modes of display are indicated by I, II, III etc. in Fig. 1.

The digital output image 160 may be displayed on a display 132, which is integral with the medical observation device 100. For example, the display 132 may be integrated in an ocular or eyepiece 104 of the medical observation device 100.

The digital output image 160 is preferably generated in real-time, i.e. a digital output image 160 is preferably generated at the same rate at which the input image data 120 are recorded or refreshed. If the digital input images 130 are part of a video stream or sequence, the digital output image 160 is generated between two immediately successive frames.

The medical observation device 100 may comprise a direct optical path 134 from the object 106 through the objective 174 to the eyepiece 104. In such a case, the display may be a translucent display 132 located in the direct optical path 134 or the display may be projected into the direct optical path 134. A beam splitter 136 may be provided to split the light between the optical eyepiece 104 and the digital imaging system 102. In one embodiment, up to 80 % of the light may be directed to the eyepiece 104.

Alternatively, the medical observation device 100 need not have a direct optical path 134 but only display images from the integral display 132. As a further alternative, the medical observation device need not have any display at all.

The medical observation device 100 may comprise an output interface 172 to which one or more (external) displays 182 may be connected. For this, the output interface 172 may comprise standardized connectors and data transmission protocols, such as USB, HDMI, DVI, DisplayPort, Bluetooth and/or others. An external display may be a monitor, 3D goggles, oculars and the like. Any combination of external displays may be connected to output interface 172.

The computer 186 and/or the data processing device 170 is connected to the digital imaging system 102 using one or more data transmission lines 196. A data transmission line may be wired or wireless, or partly wired and partly wireless. The computer 186 and/or the data processing device 170 need not be bodily integrated in the medical observation device 100 but be physically located remote from the digital imaging system 102. For this, the digital imaging system 102 and the computer 186 and/or the data processing device 170 may be connected to a network, such as a LAN, a WLAN or a WAN, to which also at least one display 182 is connected.

According to a modification, the medical observation device 100 may be stereoscopic but comprise only two cameras, one for each stereoscopic channel. In one stereoscopic channel, the fluorescence camera 111 is used and configured to selectively record white-light reflectance, whereas in the other stereoscopic channel, the reflectance camera 110 is used. Such an arrangement provides a stereoscopic reflectance color input image if no fluorescence is used and a monoscopic white-light color input image and a monoscopic fluorescence color input image if fluorescence is used. The description above and below applies equally to this configuration.

The input image data 120 may, in one embodiment, contain at least one digital color input image 130 which contains both the reflectance signal and the fluorescence emission signal. This is the case if all fluorescence emitted by the object 106 is recorded in the at least one digital color input image.

In another embodiment, the input image data 120 may contain at least one digital monochrome input image and at least one digital color input image 130. The digital monochrome input image 130 contains either at least a part of the fluorescence emission signal or at least a part of the reflectance signal. The digital color input image 130 may contain the fluorescence emission signal if the digital monochrome color input image contains the reflectance signal, or it may contain the reflectance signal if the digital monochrome input image contains the fluorescence emission signal.

The input image data 120 may comprise a plurality of input pixels. The input pixels may be color pixels or monochrome pixels. A monochrome pixel only represents intensity, e.g. as a greyscale image. A color pixel comprises information about at least some of the color appearance parameters such as hue, lightness, brightness, chroma, colorfulness and saturation. Color pixels are recorded using color bands or, equivalently, color channels or primary colors of a color space using a digital color camera.

A color space may comprise at least three color channels. In the color space, each color channel is represented by a different color space coordinate. For conversion between different color spaces, color space transformations may be used. In a different color space, the same color is represented in different color space coordinates. Each pixel of the digital color input images 130 comprises a set of color space coordinates that together represent the color of the respective pixel. Each color band thus may be regarded as representing a color space axis and each color may be regarded as a point in color space, which is defined by the vector-i.e. the color space coordinates-pointing to this color. Adding two colors may correspond to vector addition. If one color has color space coordinates {x₁, y₁, z₁} and another color has color space coordinates {x₂, y₂, z₂} then the sum of these two colors has color space coordinates {x₁+x₂, y₁+y₂, z₁+z₂}.

In one example, the digital input images 130 or, more generally, the input image data 120, may be recorded in RGB color space using the three primary colors, color bands, or color space coordinates R, G, B. Alternatively, the digital input image 130 may be recorded in different color spaces, respectively, and/or represent multi-spectral or hyperspectral color input images. The digital input images 130 of a set of digital input images, such as the digital white-light color input image 114 and the digital fluorescence color input image 112, need not be recorded in the same color space, although this is preferred.

In RGB color space, each color is represented by triple of three color space coordinates in the form of integer numbers, wherein each integer number indicates the intensity of one of the primary colors R, G, B. For example, the most intense red is indicated by the triple [255, 0, 0]. The most intense green color is indicated by [0, 255, 0], and the most intense blue by [0, 0, 255]. Thus, RGB color space is a three-dimensional space, CMYK color space would be a four-dimensional space. A multispectral or hyperspectral color space having n color bands would correspondingly result in an n-dimensional color space, in which each color is represented by an n-tuple of color space coordinates.

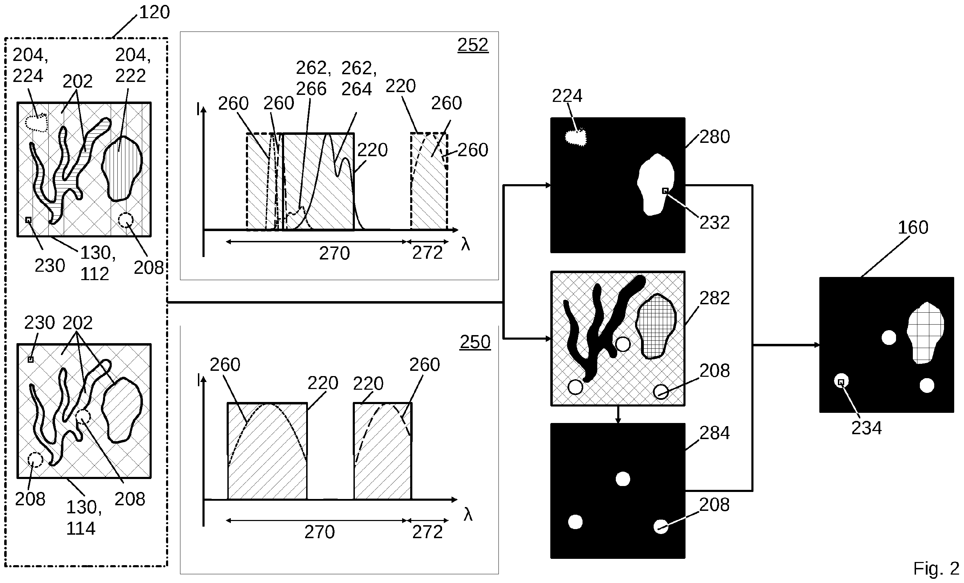

In Fig. 2, it is exemplarily shown that the input image data 120 may contain two digital input images 130, each comprising input pixels 230. The two digital input images 130 may in one instance be a digital fluorescence input image 112 and a digital reflectance input image 114as described in the context of Fig. 1.

A sample spectrum of the digital fluorescence input image 112 is indicated at reference numeral 252. The digital fluorescence input image 112 represents light that is recorded in one or more passbands 220, i.e., the spectral content represented in the digital fluorescence input image 112 may limited to one or more spectral bands, wherein each spectral band is represented by a passband 220. Each passband 220 may be contained in or contain the fluorescence emission spectrum of one or more fluorophores 116, 118.

For example, the digital fluorescence input image may contain a fluorescence emission signal 204 which comprises a first fluorescence emission signal 222 of a first fluorophore 118 and optionally a second fluorescence emission signal 224 of a second fluorophore 116. Correspondingly, the spectrum 262 of the fluorescence emission signal 204 may comprise the fluorescence emission spectrum 264 of the first fluorophore 118 and the fluorescence spectrum 266 of the second fluorophore 116. The first fluorophore may, for example, be 5-ALA. The spectrum 266 may correspond to the autofluorescence spectrum of the object 106.

The digital fluorescence image 112 may also contain at least parts of the reflectance signal 202. The reflectance signal 202 in the digital fluorescence image 112 may include the specular reflection signal 208. A sample spectrum 260 of the reflectance signal 202 is indicated in the spectrum 252.

Just by way of example, the reflectance signal 202 may contain a spectral component which may be narrowband and be located in or even confined to the NIR (near infrared) range 272 of light. Other components of the reflectance signal may be located in a separate wavelength band in the visible light range 270. These components may, for example, correspond to light in the fluorescence excitation spectrum of a fluorophore, which is used to trigger the fluorescence of the fluorophore 116. The low-frequency part of the illumination of the object used to trigger fluorescence my overlap with the highfrequency components of the fluorescence emission spectrum 260 of the respective fluorophore 116, 118. As the illumination for fluorescence excitation has a larger intensity than the fluorescence emission, the reflected illumination leaking into the passband 220 outshines the fluorescence emission and thus is part of the reflectance signal. As indicated in Fig. 2, two different excitation bands may be used to trigger the fluorescence of two different fluorophores.

A sample spectrum 250 of the digital reflectance input image 114 is also shown in Fig. 2. Preferably, the digital input images 112, 114 are recorded in complimentary, in particular, non-overlapping spectra. That is, the respective passbands 220 in the spectra 252, 250 show no or at most a minimum overlap. Preferably, they complement each other to record the entire visible spectrum 270 and potentially also the NIR range 272.

In the digital reflectance input image 114, the passbands 220 are preferably wider, so that the digital reflectance input image 114 covers a larger part of the visible light range 270 than the digital fluorescence input image 114. The wider coverage of the visible spectrum 272 in the digital reflectance input image makes sure that the digital reflectance input image 114 represents a good approximation to a white-light image and the natural colors of the object 106.

From the input image data 122, the fluorescence signal 204 and the reflectance signal 202 are extracted. For example, the fluorescence emission signal 204 and the reflectance signal 202 (if present) of the digital fluorescence image 112 may be separated from one another using spectral unmixing and/or linear transformation.

If the digital fluorescence input image 114, consists of the fluorescence emission signal, then the extracted fluorescence emission signal 280 may simply correspond to the digital fluorescence input image 114. If a plurality of digital input images 130 consists of parts of the fluorescence emission signal 130, then the extracted fluorescence emission signal 280 simply corresponds to a combination of the plurality of digital input images 130, e.g., by simple addition.

The digital reflectance image 114 already consists of part of the reflectance image 202, so the latter needs not to be extracted. However, the specular reflection signal 208 may be extracted from the digital reflectance image 114 or the reflectance signal 292, respectively.

The part of the reflectance signal 202 extracted from the digital fluorescence image 114 may be combined with the reflectance signal 202 in the digital reflectance image 114-i.e. the digital reflectance image 114 as a whole-e.g. by addition, in particular pixel-wise addition.

In this way an extracted fluorescence emission signal 280, an extracted reflectance signal 282 and an extracted specular reflectance signal 284 may be obtained. Each of these extracted signals 280, 282, 284 represents an image of the object 106 in different wavelengths (fluorescence emission signal and reflectance signal) and intensities or polarization (specular reflection signal) having pixels 232. The extracted signals 280, 282, 284 may correspond to color images or to monochrome images. In case of a monochrome extracted signal 280, 282, 284, each pixel 232 of the extracted signal represents only the intensity of the fluorescence emission at the location of the object 106 that corresponds to the location of the pixel 230.

The specular reflection signal 284 is obtained using the specular reflection extraction routine 146 (Fig. 1). Depending on the specific specular reflection extraction routine 146, it may not be necessary to first extract the reflectance signal 202 from the input image data 120 and then to extract the specular reflection signal 208 from the extracted reflectance signal 282. Instead, the specular reflection signal may be obtained directly from the input image data 122.

The digital output image 160, which may comprise output pixels 234, is obtained from, e.g., combining the extracted fluorescence emission signal 280 and the extracted specular reflection signal 284. Here, the signal/image combination routine 144 may be applied to the extracted fluorescence emission signal 280 and the extracted specular reflection signal 284. The signal/image combination routine may involve a linear transformation, such as an addition or a multiplication with a transformation matrix, of the signals 280, 284. For example, a union set formed from the union of the color space coordinates of the input pixels 232 of the signals 280, 284 at each pixel 232 may be multiplied with a transformation matrix. This can be done with color or with monochrome pixels 232.

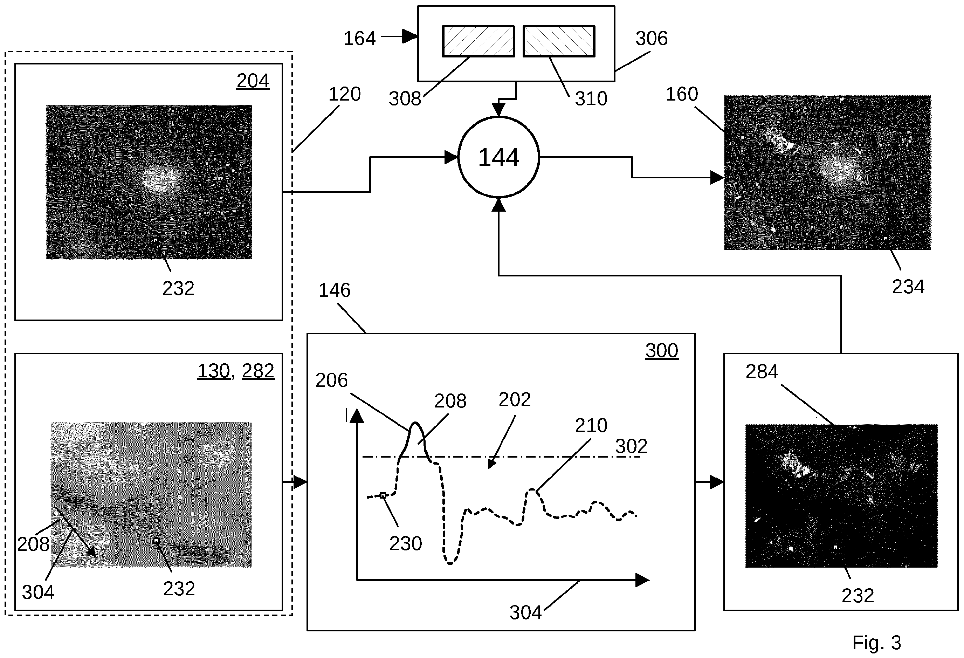

In Fig. 3, it is shown how the specular reflection signal 206 may be extracted and combined with the extracted fluorescence emission signal 204.

The digital input image 130 or the extracted reflectance signal 202 comprises specular reflections 208. At 300, the variation of intensity I along a line 304 in the digital input image 130 or the extracted reflectance signal 282 is shown. The line 304 passes through a specular reflection 208 and consists of a series of adjacent pixels 232.

According to one example of a specular reflection extraction routine 146, all pixels 232 in the digital input image 130 or the extracted reflectance signal 282 that have an intensity I exceeding an intensity threshold 302 are considered to belong to the specular reflection signal 206. All pixels 232, which have an intensity I equal to or less than the threshold value 302 are considered to belong to the remainder of the reflectance signal 202 and are not considered in the specular reflection signal 206. The extracted specular reflection signal 284 is thus made up only of pixels 232 having an intensity I larger than the intensity threshold 302.

The signal combination routine 144 then combines the extracted specular reflection signal 284 with the extracted fluorescence emission signal 204. When the extracted fluorescence emission signal 280 and the extracted specular reflection signal 284 are combined, different colors 308, 310 may be assigned to the extracted signals 280, 284, respectively. The colors 308, 310 may be part of a set 306 of colors that may be stored in the memory 194 (Fig. 1). The assignment of the colors 308, 310 may be independent of whether the extracted signals 280, 284 are represented in color or in monochrome and may be selected depending on a user selection signal 164.

For example, the first color 308 may be assigned to the specular reflection signal 284. The first color 308 may correspond to the natural color of the illumination in which the object 106 is illuminated. The color 308, in which the object 106 is currently illuminated, e.g., by the light source 199 (Fig. 1), may also be stored in the memory 194. Thus, at each output pixel 234, the component representing the extracted specular reflection signal 284 may have a color that corresponds to an illumination of the object 106. By assigning the first color 308 to the extracted specular reflection signal 284, a more natural look may be obtained for the specular reflections. Instead of a natural color as the first color 308, a false color or a pseudocolor may be used to clearly mark the specular reflections 208.

The second color 310 may be assigned to the extracted fluorescence emission signal 280. The second color 310 may be the natural color of the fluorescence emission as perceived by human vision, or a false color. The intensity of the color 310 at an output pixel 234 may depend on the intensity of the extracted fluorescence emission signal 280 at the corresponding pixel 232. Alternatively a false color or a pseudocolor may be assigned to the extracted fluorescence emission signal 280.

The intensity of the first and/or second color 308, 310 in the extracted signals 280, 284 as contained in the digital output image 160 may depend on the intensity of the respective pixels 232. If using pseudo-color, the hue of the color 308, 310 may depend on the intensity.

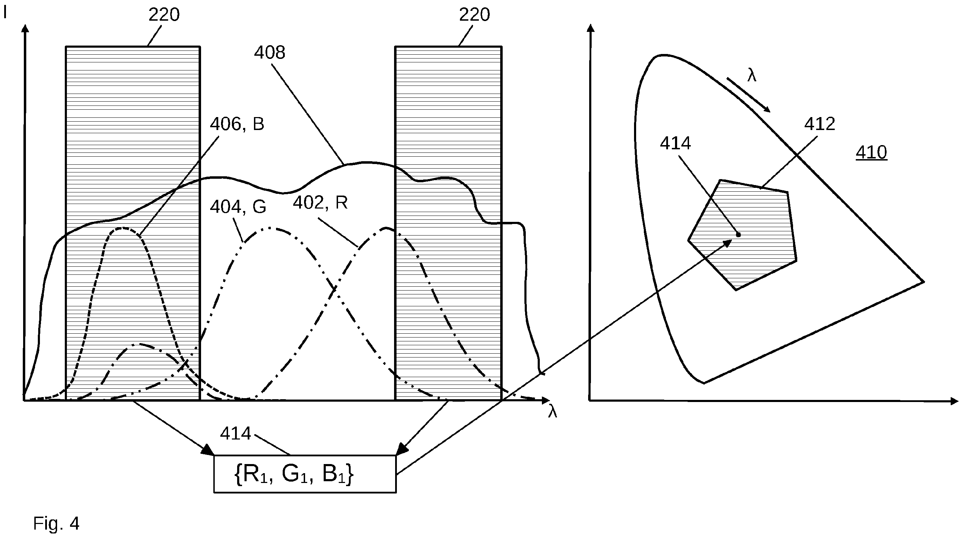

In addition to, or instead of extracting the specular reflection signal 206 by using an intensity threshold 302, the specular reflection signal may be extracted or separated from the input image data 120 by its spectral content. This is explained with reference to Fig. 4.

In Fig. 4, a sample reflectance spectrum 408 at a location of the object is shown, which location corresponds to a pixel 230, 232 is shown. At the pixel 230, 232, the spectrum 408 is recorded after passing through one or more passbands 220. Thus, at the pixel 230, 232 a color 414 is recorded corresponding to color space coordinates {R₁, G₁, B₁}.

Each color space coordinate of a pixel may be recorded by a different sensor. Each sensor records the light in a specific color band. The spectral sensitivity spectral sensitivities of these sensors vary across the wavelength as is indicated by the spectral sensitivity curves 402, 404 and 406. In the case of an RGB image, the spectral sensitivity curve 406 may quantitatively represent the B color space coordinate, the spectral sensitivity curve 404 may quantitatively represent the G coordinate, and the spectral sensitivity curve 402 may quantitatively represent the R coordinate. The light in the at least one passband 220 reaching the sensors of a pixel will thus most likely trigger a response of all three sensors as can be seen from the sensitivity curves 402, 404 and 406, resulting in the color space coordinates R₁, G₁, B₁.

A specular reflection is a total reflection of the light illuminating the object 106. Therefore, the color of a specular reflection corresponds to the color of illumination. This can be used to identify a specular reflection 208 using the color of a pixel: if the color 414 corresponds to the (known) color of the illumination spectrum the input pixel 232 is deemed to represent a specular reflection.

To allow for small deviations in the color 414, a region 412 in a color space 410 may be predetermined. The region 412 may contain the colors 414 that can be expected by a pixel 230, 232 which corresponds to a location on the object 106, where the illumination having spectrum 408 is totally reflected. The region 412 may be obtained by calibration.

Thus, if the color 414, e.g., {R₁, G₁, B₁}, of a pixel 230, 232 is contained in the region 412, then the pixel 230, 232 is considered as representing specular reflection.

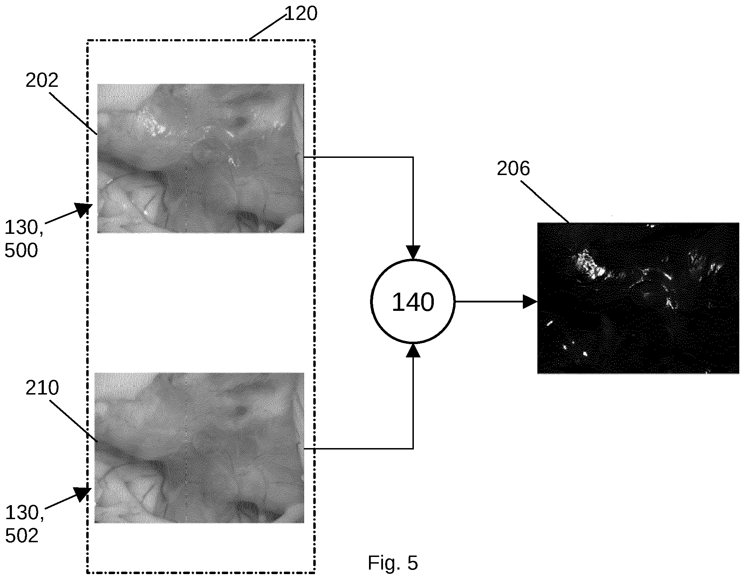

The extractions shown in Fig. 4 and 5 may be combined to even more reliably identify the specular reflection signal 206.

Another way for extracting the specular reflection signal 206 is explained in the following with reference to Fig. 5. The routine or method of Fig. 5 for extracting the specular reflection signal may be used alone or in combination with one or both of the methods or routines of Figs. 3 and 4.

According to Fig. 5, the input image data 120 comprise a reflectance signal 500 and a second reflectance signal 502, which both can be viewed as parts of the reflectance signal 202. The first reflectance signal 500 may be contained in a digital input image 130, which may only comprise the first reflectance signal 500. The second reflectance signal 500 may be contained in another digital input image 130, which may only comprise the second reflectance signal 502. The first and the second reflectance signals 500, 502 may alternatively be components of a single digital input image 130, e.g. forming two image channels of a single digital input image 130.

The first and/or second reflectance signals 500, 502 may be color or monochrome. digital input images. The first reflectance signal 500 may represent a reflectance image of the object of reflected light having a polarization that is different from the polarization represented in the second reflectance signal 502. The first reflectance signal 500 may comprise the specular reflection signal 206, whereas the second reflectance signal 502 may not comprise the specular reflection signal 206, i.e. correspond to the reflection signal 210 without the specular reflection signal.

For example, the first reflectance signal 500 may be recorded by a camera 108 having no polarization filter or a polarization filter which is different or differently oriented from a polarization filter of another camera 108 recording the second reflectance signal 502. The polarization filter in one embodiment may be part of the observation filter assembly 187, in particular of the observation filter set 188 and/or the fluorescence filter set 190. The first and second reflectance signals 500, 502 may in an alternative configuration also be recorded by a single camera 108, which may include an on-chip polarizer.

The specular reflection signal 206 may in this case be extracted by combining the first and second reflectance signals 500, 502, in particular by subtracting them. Such a subtraction may be part of the separation or extraction routine 140, in particular the specular reflection extraction routine 146.

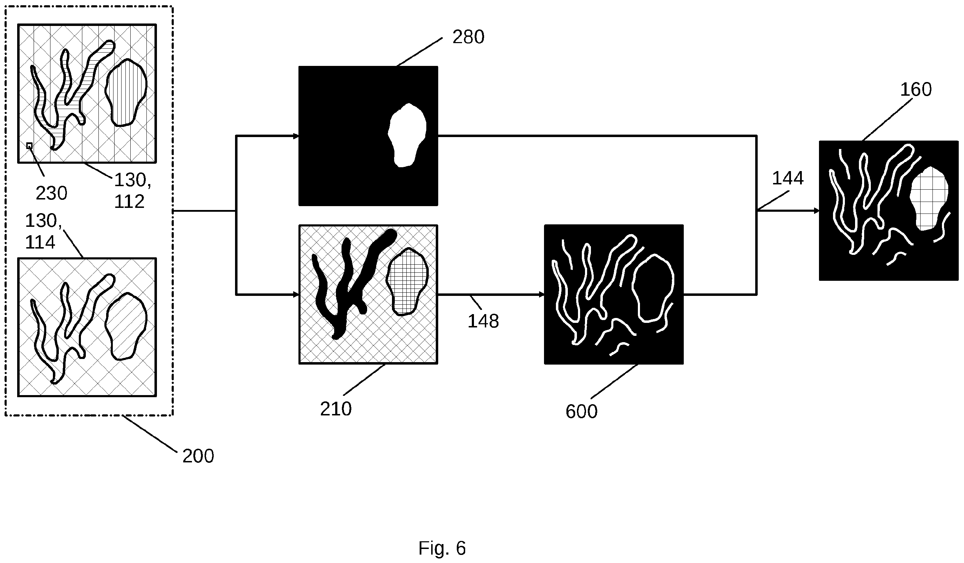

In another embodiment shown in Fig. 6, an edge detection signal 600 is generated from the reflectance signal 210 without the specular reflection signal. Although an edge detection signal 600 may also be generated from the reflectance signal 202 comprising the specular reflection signal, this is not preferred, as the specular reflections 208 will lead to additional edges which do not reflect the structure and/or topography of the object.

The edge detection signal 600 may be obtained by applying an edge detection routine 148 to the reflectance signal 210. The edge detection routine may be based on the first and/or second order derivatives of one or more color channels or of brightness, it may be based on phase stretch transforms and/or other edge detection routines typically contained in image processing libraries. The edge detection routine may be anisotropic or isotropic.

The edge detection signal 600 may be normalized and combined with the fluorescence emission signal 204, in particular the extracted fluorescence emission signal 280 for obtaining the digital output image 160.

The edge detection signal is then combined with the extracted fluorescence emission signal 280 in the same manner as the extracted specular reflection signal 284, described above, to result in the digital output image 160.

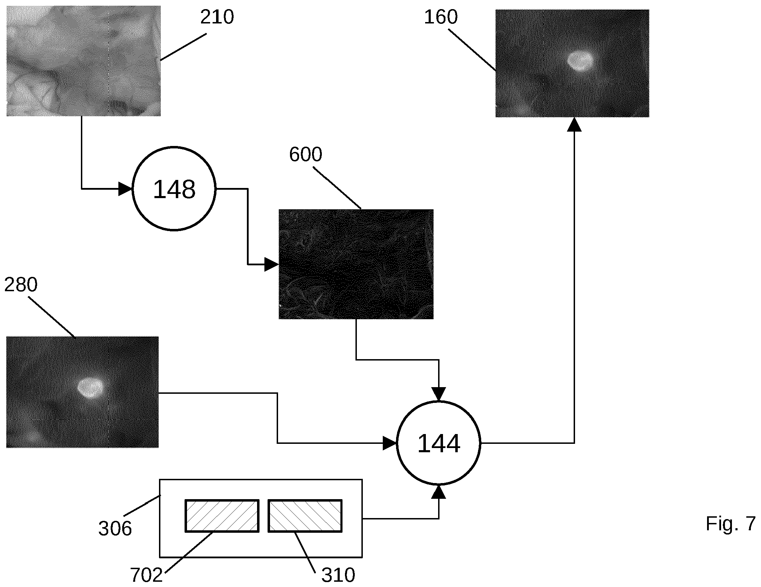

As shown in Fig. 7, at least one of the extracted fluorescence emission signals 280 and the edge detection signal 600 may be colored when the digital output image 160 is generated.

The extracted edge detection signal 600 may be colored in a color 702. The color 702 may be the natural color at the corresponding input pixel of the digital input image 130 to which the edge detection was applied. Alternatively, a false color or a pseudocolor may be applied. Alternatively, the extracted edge detection signal 600 may be normalized and converted to greyscale by the signal/image combination routine 144.

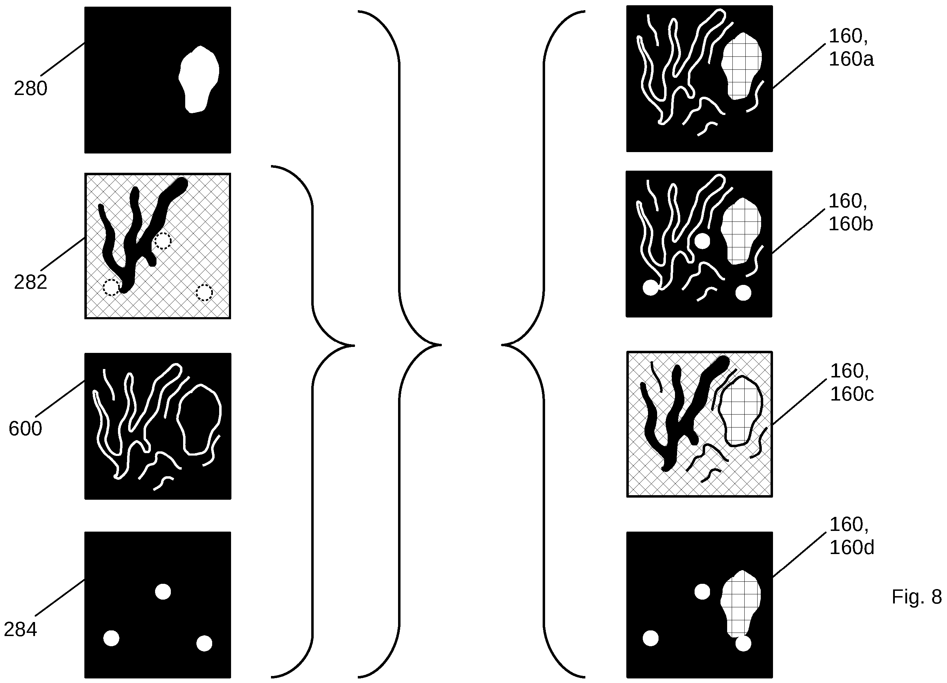

In Fig. 8, it is shown that the extracted fluorescence emission signal 280 may be combined with at least one signal of the group of signals containing the extracted reflectance signal 282 (with or without the specular reflection signal), the edge detection signal 600 and the extracted specular reflection signal 284. Any of the possible combinations results in a different output image 160a, 160b, 160c, 160d. For example, the digital output image 160a is generated from a combination of (only) the extracted fluorescence emission signal and the edge detection signal. A digital output image 160b may be generated from a combination of (only) the extracted fluorescence emission signal 280, the extracted specular reflection signal 284 and the edge detection signal 600. Another digital output image 160c may be generated from a combination of (only) the extracted digital reflectance signal 282 without the specular reflection signal, the edge detection signal 600 and the extracted fluorescence emission signal 280. Another digital output image 160d may be generated from a combination of (only) the extracted emission signal 280 and only the specular reflection signal 284.

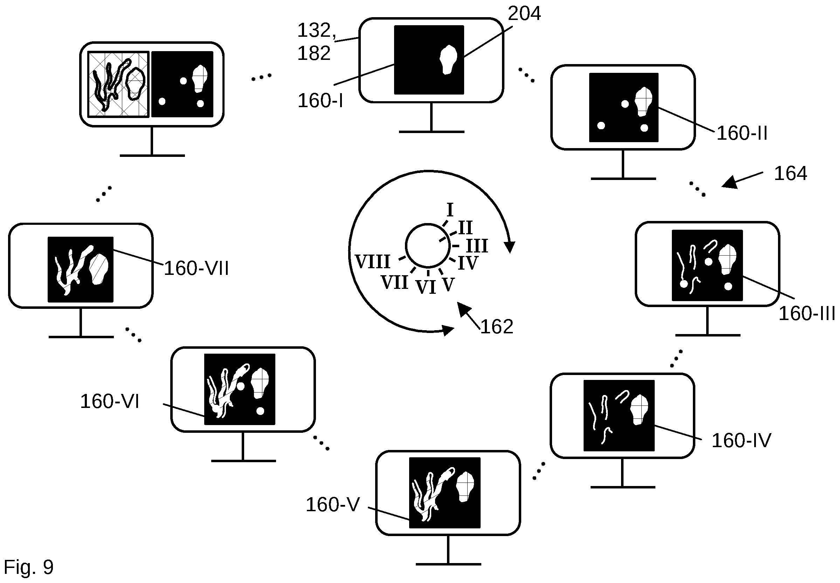

The user may select, which of the digital output images 160a, 160b ... should be generated by operating the mode selector 162 (Fig. 1). Moreover, the user may determine the relative intensity of the various signals 280, 282, 600, 284 in the respective digital output image 160a, 160b ... i.e., dim one or more signals with respect to one or more of the other signals contained in the digital output image 160. This is now explained with reference to Fig. 9.

In Fig. 9, the mode selector 162 is by way of example shown as a rotatable knob, which may be physical or a widget. The mode selector 162 has a plurality of positions I, II, ..., VIII. The number of positions depends on the application and implementation and may be larger or smaller than shown. Each of the positions I, II, ..., VIII corresponds to a different digital output image 160-I, 160-II, ..., 160-VIII, which, e.g., may correspond to the digital output images 160a, 160b, ..., 160d of Fig. 8. The positions I, II, ..., VIII may be discrete or there may be intermediate positions between the positions I, II, ..., VIII or a continuous transition between the positions I, II, ..., VIII is possible.

In each of the different digital output images 160-I, 160-II ... a different combination of extracted signals 282, 600, 284, 280 is contained. By changing the state of the mode selector 162, a user selection signal 164 (Fig. 1) is generated and sent to the digital processing unit 170, which then combines the at least one signal 282, 600, 284, 280 at the relative intensity predetermined by the respective position of the mode selector 162.

For example, the digital output image 160-I may consist only of the extracted fluorescence emission signal 280. The digital output image 160-II may consist of a combination of the extracted fluorescence emission signal 280 and the extracted specular reflection signal 284 at their respective maximum intensity. In the intermediate positions located between the positions I and II, the data processing device 170 may be configured to increase the intensity of the extracted specular reflection signal 284 relative to the extracted fluorescence emission signal 280 until position II is reached. Of course, this can also be done with additional input devices, such as widgets or physical buttons or dials, which are individually assigned to a signal. In the latter configuration, the relative intensity of each signal may be adjusted individually.

At position III, the data processing device 170 is triggered by the user selection signal 164 to generate the digital output image 160-III which contains a combination of the edge detection signal 600, the extracted specular reflection signal 284 and the extracted fluorescence emission signal. The data processing device 170 may be configured to generate the digital output image 160-III if it receives a user input signal 164, which corresponds to position III of the mode selector 162. The intensity of the edge detection signal 600 may increase, the closer the mode selector 162 is to position III.

The digital output image 160-IV may correspond to a combination of only the extracted fluorescence emission signal 280 and the edge detection signal 600. The closer the position of the mode selector 162 to position V, the smaller the intensity of the specular reflection signal 284 in the digital output image 160. This scheme may then continue by adding the extracted reflectance signal 282 without the specular reflection signal with increasing intensity when the mode selector progresses from position IV to position VI and the digital output image 160 morphs from the digital output image 160-IV to 160-V.

Next, the extracted specular reflection signal 284 may be blended in when moving the mode selector 162from position V to position VI. Further, the fluorescence emission signal 204 may slowly be blended out until it is not contained anymore in the digital output image 160-VII and only the extracted reflectance signal 282 with or without the specular reflections is contained in the digital output image 160-VII. Of course, the data processing device 170 may be configured to generate more than one digital output image 160 at the same time and to display them simultaneously. Then an adjustment of the digital output images 160 as described is possible for each of the simultaneously generated digital output images 160.

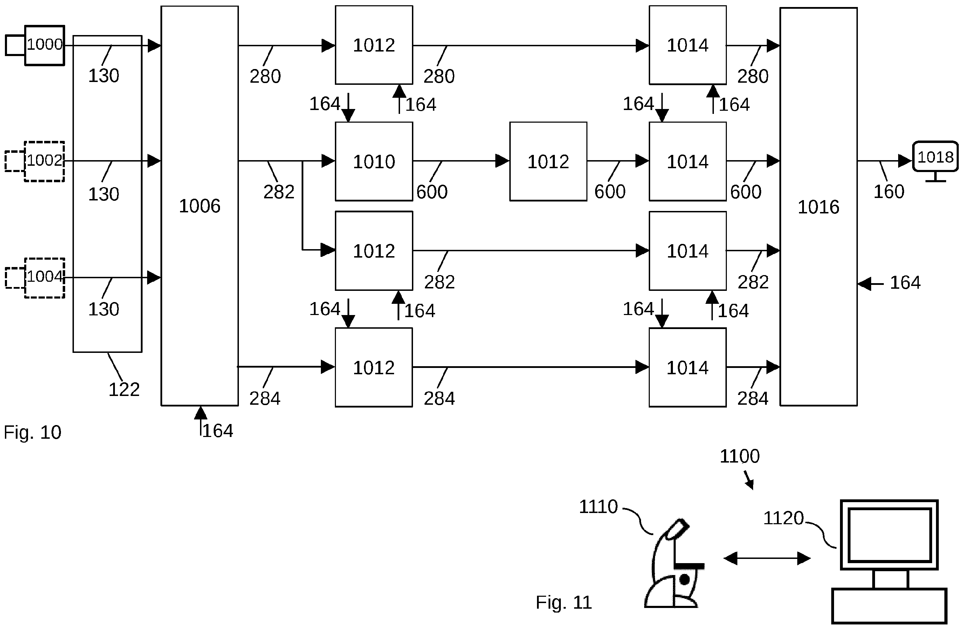

Fig. 10 provides an overview of the method steps that are performed, for example by the data processing device 170, for generating the digital output image 160.

At step 1000 and optional steps 1002 and 1004, the input image data 122 are recorded. For example, at step 1000, a single digital color input image 130 may be recorded, which contains both the reflectance signal with a specular reflectance signal and the fluorescence emission signal. At step 1002 and/or step 1004 additional input images 130 may be recorded. As described above, a digital input image 130 may contain any combination of the reflectance signal and the fluorescence emission signal or parts thereof.

At step 1006, the fluorescence emission signal and/or the reflectance signal are extracted. The scope of the term extraction also encompasses a situation where a single digital input image 130 consists of the signal to be extracted. In this case, the extraction simply involves using the respective digital input image as the signal to be extracted. The step 1006 may also encompass extracting the specular reflection signal either from the extracted reflectance signal 282 or directly from the input image data 122.

As a result of step 1006, the extracted fluorescence signal 280 and the extracted specular reflection signal 284 and optionally the extracted reflectance signal 282 are obtained. Which of these signals is extracted may be determined by the user selection signal 164, as was explained above.

At step 1010, the edge detection signal 600 may be computed from the extracted reflectance signal 282, which preferably does not contain the specular reflection signal 284. Whether the edge detection signal 600 is computed may depend on the user selection signal.

At step 1012, any of the extracted signals 280, 282, 284 or 600 may be colored, e.g., as described above.

At step 1014, the relative intensity of the respective extracted and/or the color of the signals 280, 600, 282, 284 may be changed depending on the user input signal 164. Coloring is best done when the respective signal has been normalized in a step before, e.g., in at step 1006 or at step 1012.

At step 1016, the digital output image 160 is generated from a combination of the extracted fluorescence emission signal 280 with one or more of the extracted signals 600, 282, 284. Which signals are combined in the digital output image 160 may be determined by the user selection signal.

The digital output image 160 may then be displayed at step 1018.

Some embodiments relate to a microscope comprising a system as described in connection with one or more of the Figs. 1 to 10. Alternatively, a microscope may be part of or connected to a system as described in connection with one or more of the Figs. 1 to 10. Fig. 11 shows a schematic illustration of a system 1100 configured to perform a method described herein. The system 1100 comprises a microscope 1110 and a computer system 1120. The microscope 1110 is configured to take images and is connected to the computer system 1120. The computer system 1120 is configured to execute at least a part of a method described herein. The computer system 1120 may be configured to execute a machine learning algorithm. The computer system 1120 and microscope 1110 may be separate entities but can also be integrated together in one common housing. The computer system 1120 may be part of a central processing system of the microscope 1110 and/or the computer system 1120 may be part of a subcomponent of the microscope 1110, such as a sensor, an actor, a camera or an illumination unit, etc. of the microscope 1110.

The computer system 1120 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 1120 may comprise any circuit or combination of circuits. In one embodiment, the computer system 1120 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 1120 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 1120 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 1120 may also include a display device, one or more speakers, and a keyboard and/or controller, which can include a mouse, trackball, touch screen, voice-recognition device, or any other device that permits a system user to input information into and receive information from the computer system 1120.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, afield programmable gate array may cooperate with a microprocessor in order to perform one of the methods described herein. Generally, the methods are preferably performed by any hardware apparatus.

### Reference Numerals

- 100: medical observation device
- 101L: stereoscopic subassembly for the left channel
- 101R: stereoscopic subassembly for the right channel
- 102: digital imaging system
- 104: eyepiece
- 106: object
- 107: biological tissue
- 108: digital camera
- 110: digital reflectance camera
- 111: digital fluorescence camera
- 111a: second digital fluorescence camera
- 112: digital fluorescence input image
- 114: digital reflectance input image
- 116: fluorophore, e.g. fluorophore naturally contained in object
- 118: fluorophore, e.g. fluorophore artificially added to object
- 120: input image data
- 130: digital input image
- 132: internal display
- 134: direct optical path
- 136: beam splitter
- 140: separation or extraction routine
- 142: edge detection routine
- 144: signal/image combination routine
- 146: specular reflection extraction routine
- 148: edge detection routine
- 160: digital output image
- 160a, 160b,: ... different digital output images
- 160-I, 160-II,: ... different digital output images
- 162: mode selector
- 164: user selection signal
- 166: arrow
- 168: on-chip polarizer
- 170: data processing device
- 172: output interface
- 174: objective
- 176: color-separation assembly
- 178: illumination assembly
- 179: illumination filter
- 180: illumination beam splitter
- 182: display
- 184: field of view
- 186: computer
- 187: observation filter assembly
- 188: observation filter set
- 190: fluorescence filter set
- 192: dichroic beam splitter
- 194: memory
- 196: data transmission line
- 198: illumination light
- 199: light source
- 202: reflectance signal
- 204: fluorescence emission signal
- 206: specular reflection signal
- 208: specular reflection
- 210: reflectance signal without specular reflection signal
- 212: optional component of reflectance signal in NIR
- 214: component of reflectance signal
- 220: passband
- 222: fluorescence emission signal of one fluorophore
- 224: fluorescence emission signal of another fluorophore
- 230: input pixel
- 232: pixel
- 234: output pixel
- 250: spectrum of digital reflectance input image
- 252: spectrum of digital fluorescence input image
- 260: spectrum of reflectance signal
- 262: spectrum of fluorescence emission signal 204
- 264: spectrum of fluorescence emission 222
- 266: spectrum of fluorescence emission signal 224
- 270: visible light
- 272: NIR range
- 280: extracted fluorescence emission signal
- 282: extracted reflectance signal
- 284: extracted specular reflection signal
- 300: intensity distribution along line 304
- 302: intensity threshold
- 304: line in reflectance signal/digital input image
- 306: set of colors
- 308: (first) color
- 310: (second) color
- 402: sensitivity curve of a first color space coordinate
- 404: sensitivity curve of a second color space coordinate
- 406: sensitivity curve of a third color space coordinate
- 408: spectrum of light reflected off object 408
- 410: color space
- 412: set of colors/region in color space
- 414: color of pixel/specular reflection
- 500: first reflectance signal
- 502: second reflectance signal
- 600: edge detection signal
- 702: color for edge detection signal
- 1000: recording a first digital input image
- 1002: recording a second digital input image
- 1004: recording a third color input image
- 1006: extracting/separating fluorescence emission signal and/or reflectance signal
- 1010: edge detection
- 1012: colorization of one or more signals
- 1014: adjusting/changing relative intensities
- 1016: combining the signals/greeting the digital output image
- 1018: displaying the digital color output image
- 1100: system, medical observation device
- 1110: microscope
- 1120: computer system
- B, B₁,: color space coordinate
- G, G₁: color space coordinate
- I: intensity
- R, R₁: color space coordinate
- λ: wavelength

## Claims

1. Data processing device (170) for a medical observation device (100), such as a microscope or an endoscope, for observing an object (106) which contains at least one fluorophore (116, 118),
wherein the data processing device (170) is configured:
- to access input image data (120) representative of an image of the object (106), the input image data containing
= a reflectance signal (202), the reflectance signal being representative of light (198) reflected off the object, and
= a fluorescence emission signal (204), the fluorescence emission signal being representative of fluorescence emitted by the object;
- to apply an edge detection routine (148) to the reflectance signal (202) to generate an edge detection signal (600); and
- to generate a digital output image (160) from a combination of the edge detection signal and the fluorescence emission signal.

2. Data processing device (170) according to claim 1,
wherein the digital output image (160) is a color image and the data processing device is configured:
- to assign at least one color to the fluorescence emission signal (204) in the digital output image (160).

3. Data processing device (170) according to claim 1 or 2,
wherein the data processing device is configured:
- to change the ratio of the intensities of the edge detection signal (600) and the fluorescence emission signal (204) in the digital output image (160) depending on a user selection signal (164).

4. Data processing device (170) according to any one of claims 1 to 3,
wherein the data processing device is configured:
- to generate the digital output image (160) from a combination of the edge detection signal (600), the reflectance signal (202) and the fluorescence emission signal (204).

5. Data processing device (170) according to any one of claims 1 to 4,
wherein the reflectance signal (202) contains a specular reflection signal (206), the specular reflection signal being representative of specular reflection off the object; and
wherein the data processing device is configured to:
- to extract the specular reflection signal.

6. Data processing device (170) according to claim 5,
wherein the data processing device (170) is configured:
- to generate the digital output image (160) from a combination of the extracted specular reflection signal (206), the edge detection signal (600) and the fluorescence emission signal (204).

7. Data processing device (170) according to claim 6,
wherein the data processing device is configured to generate the digital output image (160) from a combination of the fluorescence emission signal (204) and at least one signal selected, depending on a user selection signal (164), of the group of signals containing:
- the reflectance signal (202);
- the specular reflection signal (206); and
- the reflectance signal (210) without the specular reflection signal.

8. Data processing device (170) according to claim 7,
wherein the data processing device (170) is configured to change the ratio of intensities of the at least one selected signal and the fluorescence emission signal (204) in the digital output image (160) depending on the user selection signal (164).

9. Data processing device (170) according to any one of claim1 to 8,
wherein the input image data (120) comprise a digital input color image (130, 112) which contains at least a part of the fluorescence emission signal (204) and at least a part of the reflectance signal (202); and
wherein the digital data processing device (170) is configured to separate the reflectance signal contained in the digital input color image (112) from the fluorescence emission signal (204) contained in the digital input color image (112).

10. Data processing device (170) according to claim 9,
wherein the input image data (120) comprise an additional digital input image (130) which contains an additional part of the reflectance signal (202); and
wherein the data processing device is configured
- to generate the reflectance signal (202) in the digital output image (160) from the part contained in the digital input color image (130, 112) and the additional part contained in the additional digital input image (130).

11. Medical observation device (100), such as a microscope or an endoscope, for observing an object (106) which contains at least one fluorophore (116, 118),
wherein the medical observation device (100) comprises:
- a data processing device (170) according to any one of claims 1 to 10;
- at least one camera (110, 111, 111a), being adapted to record the input image data (120).

12. Computer-implemented method for processing input image data (120) in a medical observation device (100), such as a microscope or endoscope,
the method comprising the steps of:
- accessing the input image data (120), representative of an image of the object (106), the input image data containing
= a reflectance signal (202), the reflectance signal being representative of light (198) reflected off the object, and
= a fluorescence emission signal (204), the fluorescence emission signal being representative of fluorescence emitted by the object;
- applying an edge detection routine (148) to the reflectance signal (202) to generate an edge detection signal (600);
- assigning at least one pseudo-color (308) to the fluorescence emission signal; and
- generating a digital output image (106) from a combination of the edge detection signal and the fluorescence emission signal.

13. Method for using a medical observation device,
the method comprising the steps of
- recording input image data (120); and
- carrying out the computer implemented method of claim 12.

14. A computer program product computer-readable medium comprising instructions, which, when the program is executed by a computer, cause the computer to carry out the method of claim 12.

15. A computer-readable medium comprising instructions, which, when the program is executed by a computer, cause the computer to carry out the method of claim 12.
